(19) Europäisches Patentamt
European Patent Office
Office européen des brevets

(11) EP 0 716 074 B1

(12) FASCICULE DE BREVET EUROPEEN

(45) Date de publication et mention de la délivrance du brevet: **04.03.1998 Bulletin 1998/10**

(51) Int. Cl.$^6$: **C07C 67/00**, C07C 69/63

(21) Numéro de dépôt: **95402700.9**

(22) Date de dépôt: **30.11.1995**

(54) **Procédé de préparation d'halogénodifluoroacétates d'alkyle**

Verfahren zur Herstellung von Halogendifluoressigsäurealkylester

Process for the preparation of alkylic halogenodifluoroacetates

(84) Etats contractants désignés:
**AT BE CH DE DK ES FR GB GR IE IT LI LU NL PT SE**

(30) Priorité: **05.12.1994 FR 9414589**

(43) Date de publication de la demande:
**12.06.1996 Bulletin 1996/24**

(73) Titulaire: **ELF ATOCHEM S.A.**
**92800 Puteaux (FR)**

(72) Inventeurs:
- **Drivon, Gilles**
**F-69850 Saint Martin en Haut (FR)**
- **Wattier, Alain**
**F-69390 Vernaison (FR)**
- **Gillet, Jean-Philippe**
**F-69530 Brignais (FR)**
- **Ruppin, Christophe**
**F-69310 Pierre-Benite (FR)**

(56) Documents cités:
- **Aucun document pertinent relevé**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.15.12/3.4

## Description

La présente invention concerne un procédé de préparation directe d'halogénodifluoroacétates d'alkyle par réaction de 1,1-difluorotétrahalogènoéthanes sur un alcool.

Les halogénodifluoroacétates d'alkyle sont des intermédiaires de synthèse de produits pharmaceutiques et phytosanitaires.

De nombreuses méthodes ont été décrites pour obtenir ces halogénodifluoroacétates d'alkyle.

Le plus souvent, elles font appel à la réaction d'un alcool sur un acide halogénodifluoroacétique, ou de préférence sur les fluorures et les chlorures correspondants.

Ces halogénures (fluorure ou chlorure) d'halogénodifluoroacétyle peuvent être obtenus selon des techniques très variées.

Le brevet US 5259 938 décrit un procédé de préparation de chlorure d'ω-halogé-nodifluoroacétyle $M(CF_2)_nCOCl$, avec M = F ou Cl et n allant de 1 à 10, par oxydation photochimique en présence de chlore de composés de formule $M(CF_2)_n\ CH_xCl_y$ avec $x = 1$ ou 2 sachant que $x + y = 3$ .

Dans le Journal of Organic Chemistry, 33 (2) p. 816-9 (1968) on décrit un procédé d'accès au chlorure de bromodifluoracétyle qui comporte les étapes suivantes :

$$CF_2 = CF_2 + NaOCH_3 \xrightarrow{THF} CF_2 = C(F)(OCH_3) \xrightarrow{Brome} \rightarrow CF_2BrCFBrOCH_3$$

$$CF_2BrCFBrOCH_3 + 2ClSO_3H \rightarrow CF_2BrC(O)Cl$$

Le rendement final en $CF_2BrC(O)Cl$ par rapport au tétrafluoroéthylène $C_2F_4$ est inférieur à 30%.

Les fluorures de difluorohalogènoacétyles peuvent être obtenus également à partir de $C_2F_4$.

Notamment, le fluorure de bromodifluoacétyle peut être obtenu selon les étapes ci-après décrites dans la demande de brevet japonais publiée sous le n° JP 82 40434 :

$CF_2BrCF_2Br$ (obtenu selon $CF_2 = CF_2 + Br_2$) + $SO_3$ (ou $HSO_3F$) donne un intermédiaire contenant le groupement $BrCF_2CF_2OSO_2$—.

Cet intermédiaire est chauffé avec $H_2SO_4$ ou KF/sulfolane et conduit au fluorure de bromodifluoroacétyle $CF_2BrC(O)F$.

Les méthodes les plus citées consistent cependant à réaliser une hydrolyse sulfurique, en présence de sels mercuriques, de 1,1-difluorotétrahalogènoéthanes tels que : $CF_2BrCFClBr$, $CF_2ClCCl_3$.

Ainsi MOREL D. & DAWANS F. (Tetrahedron, 33 (12) pp 1445-7) mentionnent que le 1,2-dibromochlorotrifluoroéthane obtenu par bromation du chlorotrifluoroéthylène est hydrolysé en milieu oléum en présence d'HgO selon la réaction :

$$CF_2BrCFClBr \xrightarrow[HgO]{oléum} CF_2\ BrC(O)F$$

Avec un oléum à 30-40 %, la quantité de HgO utilisée pour activer la réaction est de l'ordre de 1 % en poids par rapport à $CF_2BrCFClBr$. Si la concentration de l'oléum est supérieure à 60 %, l'oxyde de mercure peut être supprimé.

Le brevet DE 1020970 décrit la préparation de $CF_2ClC(O)Cl$ selon une méthode analogue qui consiste à traiter $CF_2ClCClBr_2$ avec un oléum en présence de $HgSO_4$ à une température voisine de 50° C selon la réaction :

$$CF_2ClCClBr_2 + \text{oléum } (65\ \%\ SO_3) \xrightarrow[50\ -\ 60°C]{HgSO4} SO_2 + CF_2ClC(O)Cl$$

Le $CF_2ClC(O)Cl$ peut être purifié par chloration catalytique en phase gazeuse puis séparé par distillation fractionnée.

Notons également que l'on peut partir des oxydes d'oléfines perhalogénés tels que l'oxyde de tétrafluoroéthylène ou

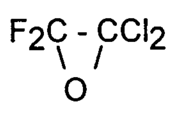

Le brevet EP 380 129 décrit la préparation de $CF_2BrC(O)F$ selon la réaction :

$$\begin{array}{l} F_2C - CF_2 + Et_3SiBr \xrightarrow[\text{- 196° C}]{\text{charbon activé}} CF_2BrC(O)F \\ \quad\; \backslash \;\; / \\ \quad\;\; O \end{array}$$

Signalons enfin que Chang-Ming Hu et Coll. (Journal of Fluorine Chemistry, 49 (1990) p. 275-280) ont décrit une méthode assez générale qui convertit un halogénoéthane en acide correspondant en faisant réagir des quantités stoechiométriques de polyfluoroperhalogénoalcane et d'un couple redox constitué de persulfate d'ammonium et de formiate de sodium.

Ainsi le 1,1-difluorotetrachloroéthane est converti en acide chlorodifluoroacétique selon la réaction :

$$CF_2ClCCl_3 + (NH_4)_2\ S_2O_8 + HCO_2Na,\ 2H_2O \rightarrow CF_2ClCOOH$$

(rendement 66,5 % - conversion 100 %)

La réaction s'effectue à 30° C en milieu DMF avec un bullage d'air. La réaction terminée, le milieu est versé dans de l'eau et la solution fortement acide est extraite avec de l'éther. L'extrait à l'éther est neutralisé avec une solution aqueuse de $NaHCO_3$. La phase aqueuse est évaporée à sec puis le résidu ($CF_2ClCO_2Na$) est repris avec $H_2SO_4$ concentré puis distillé.

Toutes ces méthodes présentent de nombreux inconvénients. Elles utilisent le plus souvent des milieux réactionnels corrosifs (oléum - $H_2SO_4$ concentré), des catalyseurs dangereux pour l'environnement (sels de mercure) ou bien font appel à des réactions susceptibles de dégager des gaz corrosifs tels que HF. Ceci entraîne, d'une part, des appareillages spécifiques et coûteux (revêtement en PVDF ou PTFE) et d'autre part, des traitements complexes des effluents si l'on veut préserver l'environnement.

Par ailleurs, il y a lieu de noter que certaines matières premières sont d'accès difficiles ou bien de manipulation dangereuse nécessitant des appareillages très spécifiques.

Le procédé qui fait l'objet de la présente invention permet d'obtenir directement de façon simple avec des rendements élevés, à partir de réactifs aisément accessibles, les halogénodifluoroacétates d'alkyle de formule :

$$CF2\ X - \underset{\underset{O}{\|}}{C} - OR \qquad (I)$$

dans laquelle X représente un atome de fluor, de chlore, de brome ou d'iode

R représente un radical hydrocarboné aliphatique, linéaire ou ramifié ayant un nombre de carbone allant de 1 à 10, et de préférence, allant de 1 à 6 ; ce procédé étant caractérisé en ce qu'il consiste à faire réagir un 1,1-difluorotétrahalogénoéthane de formule :

$$CF_2XCY_2Z \qquad (II)$$

dans laquelle X a la même signification que dans la formule (I), Y et Z, identiques ou différents représentent un atome de brome, de chlore ou d'iode, avec un alcool ROH (III), R ayant la même signification que dans la formule (I), en présence d'oxygène et en présence de générateur de radicaux libres.

A titre d'illustration de 1,1-difluorotétrahalogènoéthanes (II) utilisables selon la présente invention, on peut citer les composés de formule :

$$CF_2BrCCl_2Br,\ CF_2ClCCl_2I,\ CF_2BrCCl_2I,\ CF_3CBr_3,\ CF_3CCl_3,\ CF_2ClCCl_3,\ CF_2ClCCl_2Br.$$

Tous ces composés sont obtenus de manière connue et ne font pas l'objet de la présente invention.

A titre d'illustration d'alcool ROH utilisables selon la présente invention on peut citer le méthanol, l'éthanol, le propanol, le n-butanol, le 2-éthyl hexanol.

Selon la présente invention, en tant que générateur de radicaux libres, on peut employer soit un initiateur chimique, soit une initiation photochimique.

Selon une première variante on effectue la réaction en présence d'initiateurs chimiques tels que les peroxydes organiques comme le peroxyde de benzoyle, le peroxyde de lauroyle, le peroxyde d'acétylcyclohexanesulfonyle, le peroxyde d'isobutyroyle, le peroxyde de dichloracétyle, le peroxyde de trichloracétyle ; les hydroperoxydes organiques comme l'hydroperoxyde de tertiobutyle, l'hydroperoxyde de tertioamyle, l'hydroperoxyde de cumène, l'hydroperoxyde de paramenthane ; les peroxydicarbonates comme le peroxydicarbonate d'éthyle, le peroxydicarbonate d'éthylhexyle, le peroxydicarbonate d'isopropyle, le peroxydicarbonate d'isobutyle, le peroxydicarbonate de cétyle, le peroxydicarbonate de cyclohexyle, le peroxydicarbonate de myristyle, le peroxydicarbonate de tertiobutylcyclohexyle ; le pernéodécanoate de tertiobutyle, le pernéodécanoate de cumyle ; le perméthoxyacétate de tertiobutyle ; le peréthoxyacétate de tertiobutyle ; les composés azoïques comme le 2,2'-azo-bis (2,4-diméthylvaléronitrile), le 1,1-azobis (cyclohexane carbonitrile), l'azobis (isobutyronitrile).

Parmi ces initiateurs chimiques on préfère utiliser les composés azoïques et, tout particulièrement, l'azobis (isobutyronitrile) désigné ci-après par AIBN.

On utilise généralement 0,01 % à 10 % molaire, et, de préférence 0,5% à 5 % molaire de (ou des) initiateur(s) chimiques(s), par rapport au 1,1-difluorotétrahalogénoéthane mis en oeuvre.

Selon une seconde variante dans laquelle le générateur de radicaux libres est une initiation photochimique, on effectue la réaction sous irradiation lumineuse de longueur d'onde $\lambda$ au moins égale à 200nm et, de préférence, comprise entre 260 nm et 800 nm.

D'une manière générale les produits de formule (I) sont préparés par mise en contact du 1,1-difluorotétrahalogénoéthane (II) avec l'alcool ROH (III) et de l'initiateur chimique, si l'on opère selon la première variante, puis le mélange ainsi obtenu est chauffé sous bullage d'air, d'oxygène ou d'oxygène dilué dans un gaz inerte tel que l'azote, l'hélium, l'argon.

Dans l'éventualité où l'on opère selon la seconde variante, le mélange constitué par les réactifs (II) et (III), sous bullage d'air, d'oxygène ou d'oxygène dilué dans un gaz inerte tel que l'azote, l'helium, l'argon est soumis à une irradiation lumineuse.

Le rapport molaire alcool ROH (III)/1,1-difluorotétrahalogénoéthane (II) est supérieur ou égal à 1 et, de préférence, inférieur à 5.

Selon un mode de réalisation préféré, dans l'une ou l'autre variante, on utilisera l'alcool ROH simultanément comme réactif et solvant. Dans ces conditions le rapport molaire (III)/(II) peut varier dans une large mesure. Il est au moins égal à 1 et, au plus égal à 30. De préférence il est compris entre 5 et 20.

On ne sortirait pas du cadre de l'invention si l'on opérait en milieu solvant non alcoolique. Ce solvant ne doit ni être réactif à l'égard des réactifs (II) et (III) ni avoir d'influence sur les produits formés (I). Il doit être également totalement inerte dans les conditions opératoires.

Selon la présente invention, la réaction s'effectue sous bullage d'air, ou d'oxygène, ou bien encore d'air enrichi en oxygène. On opère généralement à pression atmosphérique ($10^5$ Pa) mais on ne sortirait pas du cadre de l'invention si on opérerait à une pression différente.

Selon l'une ou l'autre variante, la température peut varier dans une large mesure, elle est généralement comprise entre 20° C et 150° C et, de préférence comprise entre 50° C et 100° C. Il peut être nécessaire pour maintenir la température de réaction dans les limites précédemment mentionnées de refroidir le milieu réactionnel.

La durée de réaction peut varier dans une large mesure. Notamment, si l'on opère selon la seconde variante la durée d'irradiation peut aller de 15 minutes à 80 heures.

Comme source d'irradiation on utilise tout générateur de rayonnement électromagnétique dans le domaine de longueur d'onde de 200 nm à 800 nm (lampes à vapeur de mercure, néons, lampes excimères, lasers, etc..... ).

L'invention s'applique tout particulièrement à la préparation du bromodifluoroacétate d'éthyle, du chlorodifluoroacétate d'éthyle et du trifluoroacétate d'éthyle.

Les produits obtenus sont isolés du milieu réactionnel par les moyens connus de l'homme du métier à savoir une ou plusieurs distillation pour séparer les produits obtenus des réactifs non transformés qui, si besoin est, peuvent être recyclés, notamment lorsque l'alcool ROH est utilisé à la fois comme réactif et comme solvant.

Les produits sont analysés par chromotographie en phase gazeuse et identifiés par résonance magnétique nucléaire.

Le procédé qui fait l'objet de la présente invention permet d'obtenir des halogénodifluoroacétates d'alkyle (I) directement, selon des conditions opératoires douces et ne nécessitant pas l'utilisation de matériaux spéciaux et onéreux, par simple réaction entre un 1,1-difluorotétrahalogénoéthane et un alcool. En outre, les effluents sont constitués de produits peu ou pas agressifs et peuvent être éventuellement recyclés sans purification particulière.

Les exemples qui suivent illustrent l'invention.

**EXEMPLE 1**

Dans un réacteur classique muni d'une agitation type turbine, d'un réfrigérant, d'un contrôle de température et d'un

tube plongeur pour l'arrivée de gaz, on charge 145 g d'une solution éthanolique contenant 42,5 g (soit 0,145 mole) de $CF_2BrCCl_2Br$ et 102,5g (soit 2,22 moles) d'éthanol.

Puis on ajoute 1,18g d'azobisisobutyronitrile (AIBN) ce qui correspond à une quantité molaire égale à 5 % par rapport à l'halogénoéthane mis en oeuvre.

Le milieu réactionnel est porté à 65° C sous agitation et sous bullage d'air (débit moyen 7 l/h).

Après 10 heures on observe une conversion du $CF_2BrCCl_2Br$ de 50 % et un rendement en bromodifluoroacétate d'éthyle de 42 % (dosage effectué par chromatographie en phase gazeuse).

Le bromodifluoroacétate d'éthyle a été identifié par résonance magnétique nucléaire (RMN) du proton ($H^1$), du carbone 13 ($C^{13}$) et du fluor 19 ($F^{19}$) sur un appareil Brücker type AC300 multinoyaux (fréquences pour $H^1$ = 300, 13MHz, pour $C^{13}$ = 75,47 MHz et pour $F^{19}$ = 282,4 MHz).

<u>Identification RMN de</u>

a b c d
$CF_2BrCOCH_2CH_3$
II
O

- <u>Spectre RMN du $C^{13}$</u>
  (solvant = $CDCl_3$)

  δa = 108,8 ppm
  δb = 159,5 ppm
  δc = 64,5 ppm
  δd = 13,5 ppm

- <u>Spectre RMN du $F^{19}$</u>
  (solvant = $CDCl_3$/étalon externe : acide trifluoroacétique)

  δ ($CF_2Br$) = - 16,8 ppm
  constante de couplage $J^{1}_{C\text{-}F}$ = 314 Hz
  constante de couplage $J^{2}_{C\text{-}F}$ = 31 Hz

- <u>Spectre RMN du $H^1$</u>
  (solvant = $CDCl_3$/étalon interne : tétraméthylsilane)

  δ ($C\underline{H}_2$) = 4,42 ppm
  δ ($C\underline{H}_3$) = 1,40 ppm

**EXEMPLE 2**

Dans un réacteur classique muni d'une agitation type turbine, d'un réfrigérant, d'un contrôle de température et d'un tube plongeur pour l'arrivée de gaz, on charge 150 g d'une solution éthanolique contenant 45g (soit 0,153 mole) de $CF_2BrCCl_2Br$ et 105 g (soit 2,28 moles) d'éthanol.

Puis on ajoute 0,3 g d'AIBN ce qui correspond à une quantité molaire égale à 1,2 %, par rapport à l'halogénéoéthane mis en oeuvre. Le milieu réactionnel est porté à 65° C sous agitation et sous bullage d'air (débit 7 l/h). Après 15 heures de réaction on observe une conversion du $CF_2BrCCl_2Br$ de 57 % et un rendement en bromodifluoroacétate d'éthyle de 51,3 %.

**EXEMPLE 3**

Dans le même montage que l'exemple 2, on charge 150 g d'une solution éthanolique contenant 45 g (soit 0,22 mole) de $CF_2ClCCl_3$ (F112a) et 105 g (soit 2,28 moles) d'éthanol.

Puis on ajoute 0,72 g d'AIBN soit 2 % molaire par rapport au F112a mis en oeuvre.

On obtient une conversion du F112a de 73,4 % et un rendement en chlorodifluoroacetate d'éthyle de 23 %.

Identification RMN du

a b c d

$CF_2ClCOCH_2CH_3$

||

O

- Spectre RMN du $C^{13}$

  (solvant = $CDCl_3$)

  δa = 116,9 ppm

  δb = 159,2 ppm

  δc = 64,5 ppm

  δd = 13,7 ppm

- Spectre RMN du $F^{19}$

  (solvant = $CDCl_3$/étalon externe TFA)

  δ ($C\underline{F_2}Cl$) = - 15,4 ppm

  $J^1_{C\text{-}F}$ = 300 Hz, $J^2_{C\text{-}F}$ = 34,5 Hz

- Spectre RMN du $H^1$

  (solvant = $CDCl_3$/étalon interne TMS)

  δ ($C\underline{H_2}$) = 4,4 ppm

  δ ($C\underline{H_3}$) = 1,4 ppm

**EXEMPLE 4**

Dans un réacteur en verre de 125 $cm^3$, équipé d'une agitation, sont introduits 17,5 g (60 mmoles) de $CF_2BrCCl_2Br$ et 42 g d'éthanol. Le réacteur est alors placé sous irradiation coaxiale de 4 lampes de 8 w à 365 nm (montage de type "Rayonet") et la température du milieu réactionnel est maintenue à 25° C par refroidissement externe du réacteur sous courant d'air.

Après 60 heures d'irradiation, les analyses par chromatographie en phase gazeuse du brut réactionnel révèlent qu'il reste 4,5 g de $CF_2BrCCl_2Br$ non converti et qu'il s'est formé 7,5 g de bromodifluoroacétate d'éthyle. La conversion du $CF_2BrCCl_2Br$ est de 73,8 % et le rendement molaire en bromodifluoroacétate d'éthyle est de 61,5 %.

**EXEMPLE 5**

Dans un photoréacteur à lampe centrale (lampe 15 w, λ : 365 nm), d'une contenance utile de 300 $cm^3$, on charge 310 g d'une solution éthanolique contenant 90,83 g (soit 0,31 mole) de $CF_2BrCCl_2Br$ et 219,17 g (4,76 moles) d'éthanol. Une pompe extérieure assure la circulation permanente de la solution de travail.

La température du milieu réactionnel est maintenue à température ambiante par une circulation d'eau dans la double enveloppe du réacteur.

De plus, on injecte en continu, en pied de réacteur de l'air à un débit moyen de 3 l/h.

L'irradiation est mise en service.

Après 65 heures on observe une conversion du $CF_2BrCCl_2Br$ de 58,8 % et un rendement en bromodifluoroacétate d'éthyle de 55 %.

Les composés ci-dessus sont dosés par chromatographie phase gazeuse.

**EXEMPLE 6**

Dans le même montage que dans l'exemple 5, on charge 300 g d'une solution éthanolique contenant 90 g (0,30 mole) de $CF_2BrCCl_2Br$ et 210 g d'éthanol.

Le milieu réactionnel est porté à 65° C sous un débit d'air de 7l/h.

On constate après 15 heures de réaction une conversion du $CF_2BrCCl_2Br$ de 88 % et un rendement en bromodifluoroacétate d'éthyle de 66,5 %.

**EXEMPLE 7**

Dans un réacteur classique muni d'une agitation type turbine, d'un réfrigérant, d'un contrôle de température et d'un tube plongeur pour l'arrivée de gaz, on charge 47,1 g (0,147 mole) de $CF_3CBr_3$ avec 103 g (2,23 moles) d'éthanol.

Puis on ajoute 0,3 g d'AIBN soit 1,24 % molaire par rapport à l'halogénoéthane. Le milieu réactionnel est chauffé à 65° C sous agitation et sous bullage d'air (7l/h).

Après 13h30 de réaction on obtient une conversion du $CF_3CBr_3$ de 21,7 % et un rendement en trifluoroacétate d'éthyle de 17 % par rapport à l'halogénoéthane.

**EXEMPLE 8**

Dans le même montage que l'exemple 7 on charge 45 g (0,152 mole) de $CF_2ClCCl_2I$ avec 105 g (2,28 moles) d'éthanol.

Puis on ajoute 0,5 g d'AIBN soit 2 % molaire par rapport à l'halogénoéthane, Le milieu réactionnel est chauffé à 65° C sous agitation et sous bullage d'air (7l/h).

Après 8h de réaction on obtient une conversion quasi totale du $CF_2ClCCl_2I$ et un rendement de 65 % en chlorodifluoroacétate d'éthyle par rapport à l'halogénoéthane.

**Revendications**

1. Procédé direct de préparation d'halogénodifluoroacétates d'alkyle de formule :

$$CF2\,X-\underset{\underset{O}{\|}}{C}-OR \qquad (I)$$

dans laquelle X représente un atome de fluor, de chlore, de brome ou d'iode, R représente un radical hydrocarboné aliphatique, linéaire ou ramifié ayant un nombre de carbone allant de 1 à 10, caractérisé en ce qu'il consiste à faire réagir un 1,1-difluorotétrahalogénoéthane de formule :

$$CF_2XCY_2Z \qquad (II)$$

dans laquelle X a la même signification que dans la formule (I), Y et Z, identiques ou différentes représentent un atome de chlore, de brome ou d'iode, avec un alcool ROH (III), R ayant la même signification que dans la formule (I), en présence d'oxygène et en présence de générateur de radicaux libres.

2. Procédé selon la revendication 1, caractérisé en ce que (es 1,1-difluorotétrahalogénoéthanes de formule (II) sont les composés de formule :

$CF_2BrCCl_2Br$, $CF_2ClCCl_3$, $CF_3CCl_3$, $CF_3CBr_3$ et $CF_2ClCCl_2I$.

3. Procédé selon la revendication 1, caractérisé en ce que l'alcool ROH (III) est l'éthanol.

4. Procédé selon les revendications 1 à 3, caractérisé en ce que le rapport molaire alcool ROH (III)/1,1-difluorotétrahalogénoéthane (II) est supérieur ou égal à 1 et, de préférence, inférieur 5.

5. Procédé selon l'une des revendications 1 à 3, caractérisé en ce que l'alcool ROH est utilisé simultanément comme réactif et solvant.

6. Procédé selon l'une des revendications 1 à 3 et 5, caractérisé en ce que le rapport molaire alcool ROH (III)/1,1-difluorotétrahalogénoéthane (II) est au moins égal à 1 et, au plus égal à 30.

7. Procédé selon la revendication 6, caractérisé en ce que le rapport molaire (III)/(II) est compris entre 5 et 20.

8. Procédé selon l'une des revendications 1 à 7, caractérisé en ce que le générateur de radicaux libres est un initiateur chimique.

9. Procédé selon la revendication 8, caractérisé en ce que l'initiateur chimique est un composé azoïque.

10. Procédé selon la revendication 8, caractérisé en ce que le composé azoïque est l'azobis (isobutyronitrile).

11. Procédé selon l'une des revendications 1 à 7, caractérisé en ce que le générateur de radicaux libres est une initiation photochimique.

12. Procédé selon la revendication 11, caractérisé en ce que l'initiation photochimique est une irradiation lumineuse de longueur d'onde $\lambda$ au moins égale à 200 nm.

13. Procédé selon la revendication 12, caractérisé en ce que la longueur d'onde $\lambda$ est comprise entre 260 nm et 800 nm.

14. Procédé selon l'une des revendications 1 à 13, caractérisé en ce que la température est comprise entre 20° C et 150° C.

15. Procédé selon l'une des revendications 1 à 14, caractérisé en ce que l'on opère en présence d'air.

**Claims**

1. Direct process for the preparation of alkyl halodifluoroacetates of formula:

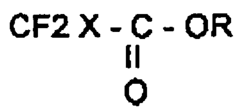

(I)

in which X represents a fluorine, chlorine, bromine or iodine atom, R represents a linear or branched aliphatic hydrocarbon radical having a carbon number ranging from 1 to 10, characterized in that it consists in reacting a 1,1-difluorotetrahaloethane of formula:

$$CF_2XCY_2Z \qquad (II)$$

in which X has the same meaning as in the formula (I) and Y and Z, which are identical or different, represent a bromine, chlorine or iodine atom, with an alcohol ROH (III), R having the same meaning as in the formula (I), in the presence of oxygen and in the presence of a free-radical generator.

2. Process according to Claim 1, characterized in that the 1,1-difluorotetrahaloethanes of formula (II) are the compounds of formula $CF_2BrCCl_2Br$, $CF_2ClCCl_3$, $CF_3CCl_3$, $CF_3CBr_3$ and $CF_2ClCCl_2I$.

3. Process according to Claim 1, characterized in that the alcohol ROH (III) is ethanol.

4. Process according to Clams 1 to 3, characterized in that the molar ratio of alcohol ROH (III) to 1,1-difluorotetrahaloethane (II) is greater than or equal to 1 and, preferably, is less than 5.

5. Process according to one of Claims 1 to 3, characterized in that the alcohol ROH is used simultaneously as reactant and solvent.

6. Process according to one of Claims 1 to 3 and 5, characterized in that the molar ratio of alcohol ROH (III) to 1,1-difluorotetrahaloethane (II) is at least equal to 1 and is at most equal to 30.

7. Process according to claim 6, characterized in that the molar ratio (III):(II) is between 5 and 20.

8. Process according to one of Claims 1 to 7, characterized in that the free-radical generator is a chemical initiator.

**9.** Process according to Claim 8, characterized in that the chemical initiator is an azo compound.

**10.** Process according to Claim 8, characterized in that the azo compound is azobis(isobutyronitrile).

**11.** Process according to one of Claims 1 to 7, characterized in that the free-radical generator comprises a photochemical initiation.

**12.** Process according to Claim 11, characterized in that the photochemical initiation comprises irradiation with light having a wavelength λ which is at least equal to 200 nm.

**13.** Process according to Claim 12, characterized in that the wavelength λ is between 260 nm and 800 nm.

**14.** Process according to one of Claims 1 to 13, characterized in that the temperature is between 20°C and 150°C.

**15.** Process according to one of Claims 1 to 14, characterized in that the reaction is carried out in the presence of air.

**Patentansprüche**

**1.** Direktes Herstellungsverfahren für Alkylhalogendifluoracetate der Formel

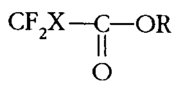 (I)

in der X ein Fluor-, Chlor-, Brom- oder Jodatom darstellt und R einen linearen oder verzweigten aliphatischen Kohlenwasserstoffrest mit 1 bis 10 Kohlenstoffen darstellt, dadurch gekennzeichnet, daß man bei dem Verfahren ein 1,1-Difluortetrahalogenethan der Formel

$$CF_2XCY_2Z \qquad (II)$$

in der X dieselbe Bedeutung wie in Formel (I) besitzt und Y und Z, identisch oder verschieden, ein Chlor-, Brom- oder Jodatom darstellen, mit einem Alkohol ROH (III), wobei R dieselbe Bedeutung wie in Formel (I) besitzt, in Gegenwart von Sauerstoff und in Gegenwart eines Stoffes, der freie Radikale erzeugt, reagieren läßt.

**2.** Verfahren nach Anspruch 1, dadurch gekennzeichnet, daß die 1,1-Difluortetrahalogenethane der Formel (II) Verbindungen der Formel $CF_2BrCCl_2Br$, $CF_2ClCCl_3$, $CF_3CCl_3$, $CF_3CBr_3$ und $CF_2ClCCl_2I$ sind.

**3.** Verfahren nach Anspruch 1, dadurch gekennzeichnet, daß der Alkohol ROH (III) Ethanol ist.

**4.** Verfahren nach einem der Ansprüche 1 bis 3, dadurch gekennzeichnet, daß das Molverhältnis von Alkohol ROH (III)/1,1-Difluortetrahalogenethan (II) größer oder gleich 1, vorzugsweise kleiner als 5, ist.

**5.** Verfahren nach einen, der Ansprüche 1 bis 3, dadurch gekennzeichnet, daß der Alkohol ROH gleichzeitig als Reagenz und als Lösungsmittel verwendet wird.

**6.** Verfahren nach einem der Ansprüche 1 bis 3 und 5, dadurch gekennzeichnet, daß das Molverhältnis von Alkohol ROH (III)/1,1-Difluortetrahalogenethan (II) mindestens 1 und höchstens 30 beträgt.

**7.** Verfahren nach Anspruch 6, dadurch gekennzeichnet, daß das Molverhältnis von Verbindung (III)/Verbindung (II) zwischen 5 und 20 liegt.

**8.** Verfahren nach einem der Ansprüche 1 bis 7, dadurch gekennzeichnet, daß die Verbindung, die freie Radikale erzeugt, ein chemischer Starter (Initiator) ist.

**9.** Verfahren nach Anspruch 8, dadurch gekennzeichnet, daß der chemische Starter eine Azoverbindung ist.

**10.** Verfahren nach Anspruch 8, dadurch gekennzeichnet, daß die Azoverbindung Azobis(isobutyronitril) ist.

11. Verfahren nach einen, der Ansprüche 1 bis 7, dadurch gekennzeichnet, daß die Erzeugung freier Radikale durch photochemische Initiation erfolgt.

12. Verfahren nach Anspruch 11, dadurch gekennzeichnet, daß die photochemische Initiation durch Bestrahlen mit Licht einer Wellenlänge $\lambda$ von mindestens 200 nm erfolgt.

13. Verfahren nach Anspruch 12, dadurch gekennzeichnet, daß die Wellenlänge $\lambda$ zwischen 260 nm und 800 nm liegt.

14. Verfahren nach einem der Ansprüche 1 bis 13, dadurch gekennzeichnet, daß die Temperatur zwischen 20 °C und 150 °C liegt.

15. Verfahren nach einem der Ansprüche 1 bis 14, dadurch gekennzeichnet, daß man in Gegenwart von Luft verfährt.